Europäisches Patentamt

European Patent Office

Office européen des brevets

(11) Publication number: **0 450 100 A1**

(12) **EUROPEAN PATENT APPLICATION**
published in accordance with Art.
158(3) EPC

(21) Application number: 90915812.3

(22) Date of filing: 26.10.90

(86) International application number:
PCT/JP90/01384

(87) International publication number:
WO 91/06565 (16.05.91 91/11)

(51) Int. Cl.5: **C07K 7/10, A61K 37/02**

(30) Priority: 26.10.89 JP 277267/89

(43) Date of publication of application:
09.10.91 Bulletin 91/41

(84) Designated Contracting States:
**CH DE FR GB IT LI**

(71) Applicant: **MEIJI SEIKA KAISHA LTD.**
**4-16 Kyobashi 2-chome**
**Chuo-ku Tokyo 104(JP)**

Applicant: **DAICEL CHEMICAL INDUSTRIES,**
**LTD.**
**1, Teppo-cho Sakai-shi**
**Osaka 590(JP)**

(72) Inventor: **YANAIHARA, Noboru**
**403-22, Kita**
**Shizuoka-shi Shizuoka 420(JP)**

(74) Representative: **Goddar, Heinz J., Dr. et al**
**FORRESTER & BOEHMERT**
**Widenmayerstrasse 4/I**
**W-8000 München 22(DE)**

(54) **PHYSIOLOGICALLY ACTIVE PEPTIDE.**

(57) New chemically synthesized peptides of the following general formulae (I), (II), (III), which have a smooth muscle relaxant activity and can increase blood flow in the blood vessel. General formula (I): H-His-Ser-Asp-Ala-Ile-Phe-Thr-Gln-Gln-Tyr-Ser-Lys-Leu-Leu-Ala-Lys-Leu-Ala-Leu-Gln-Lys-Tyr-Leu-Ala-Ser-Ile-Leu-Gly-Ser-Arg-Thr-$X-NH_2$, wherein X represents a single bond, Ser or Ser-Pro, general formula (II): H-His-Ser-Asp-Ala-Val-Phe-Thr-Asp-Asn-Tyr-Thr-Lys-Leu-Leu-Ala-Lys-Leu-Ala-Leu-Gln-Lys-Tyr-Leu-Ala-Ser-Ile-Leu-Gly-Ser-Arg-Thr-$NH_2$, general formula (III): H-His-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Ile-Leu-Asn-Ser-Arg-Thr-Ser-Pro-Pro-Pro-$NH_2$.

Rank Xerox (UK) Business Services

TECHNICAL FIELD

This invention relates to novel physiologically active peptides having an activity of relaxing smooth or unstriated muscles.

PRIOR ART

Known natural peptides having hyperkinemic action in blood vessels of mammals include VIP (vasoactive intestinal polypeptide) which is present in human intestinal tracts, and helodermin which is isolated from venom of venomous lizards. Use of these natural peptides as drugs, however, involves various problems such that VIP cannot sustain its pharmaceutical effects over any prolonged period of time, whereas helodermin is poor in the strength of its muscle-relaxing action, and others.

Helodermin is a peptide whose structure has the amino acid sequence represented by the following formula:

H-His-Ser-Asp-Ala-Ile-Phe-Thr-Gln-Gln-Tyr

-Ser-Lys-Leu-Leu-Ala-Lys-Leu-Ala-Leu-Gln-Lys-Tyr-Leu

-Ala-Ser-Ile-Leu-Gly-Ser-Arg-Thr-Ser-Pro-Pro-Pro-NH$_2$

Further, VIP is a peptide whose structure has the amino acid sequence represented by the following formula:

H-His-Ser-Asp-Ala-Val-Phe-Thr-Asp-Asn-Tyr-Thr-Arg-

Leu-Arg-Lys-Gln-Met-Ala-Val-Lys-Lys-Tyr-Leu-Asn-Ser-

Ile-Leu-Asn-NH$_2$

DISCLOSURE OF THE INVENTION

We, the present inventors, have proceeded with an investigation with a view toward providing a novel peptide having superior physiological activities to helodermin and VIP described above. As a result of our investigation, we have succeeded in chemically synthesizing three novel peptides, each of which comprises a part of the amino acid sequence of helodermin, is represented as a peptide of the below-described general formula (I) and can hence be regarded as a helodermin derivative. It has also been found that these novel peptides have physiological activity of relaxing a vascular unstriated or smooth muscle to increase the flow of blood. We, the present inventors, have further succeeded in chemically synthesizing a novel peptide composed of a combination of the amino acid sequence of VIP with a part of the amino acid sequence of helodermin, as well as a novel peptide composed of a combination of a part of the amino acid sequence of VIP with another part of the amino acid sequence of helodermin. It has also been found that the latter two novel peptides have physiological activities similar to those of the peptides of the general formula (I).

According to a first aspect of the present invention, there is thus provided a peptide represented by the following general formula (I)

H-His-Ser-Asp-Ala-Ile-Phe-Thr-Gln-Gln-Tyr-Ser-Lys

-Leu-Leu-Ala-Lys-Leu-Ala-Leu-Gln-Lys-Tyr-Leu-Ala-

Ser-Ile-Leu-Gly-Ser-Arg-Thr-X-NH$_2$          (I)

where X means a bond (-), Ser or Ser-Pro.

The peptide of the general formula (I) is derivatives which may be formed by shortening the molecule of helodermin in such a way that, among the 35 amino acid residual groups which are making up the amino acid sequence of helodermin, the 4 amino acids present at position 32 to position 35, or the three amino acids present at position 33 to position 35 or the two amino acids present at position 34 to 35 from the end N-terminal of helodermin have been deleted, independently, from the helodermin molecule; and thus the peptide of formula (I) are such shortened peptide derivatives as derived from helodermin.

The peptide of the general formula (I) includes the following three peptides (Ia), (Ib) and (Ic):

(i) Peptide of the general formula (I) where X means a bond (-), namely, the peptide represented by the following formula (Ia):

H-His-Ser-Asp-Ala-Ile-Phe-Thr-Gln-Gln-Tyr-Ser-Lys-

Leu-Leu-Ala-Lys-Leu-Ala-Leu-Gln-Lys-Tyr-Leu-Ala-

Ser-Ile-Leu-Gly-Ser-Arg-Thr-NH$_2$          (Ia)

(ii) Peptide of the general formula (I) where X means Ser, namely, the peptide represented by the following formula (Ib):

H-His-Ser-Asp-Ala-Ile-Phe-Thr-Gln-Gln-Tyr-Ser-Lys

-Leu-Leu-Ala-Lys-Leu-Ala-Leu-Gln-Lys-Tyr-Leu-Ala-

Ser-Ile-Leu-Gly-Ser-Arg-Thr-Ser-NH$_2$          (Ib)

(iii) Peptide of the general formula (I) where X means Ser-Pro, namely, the peptide represented by the following formula (Ic):

H-His-Ser-Asp-Ala-Ile-Phe-Thr-Gln-Gln-Tyr-Ser-Lys

-Leu-Leu-Ala-Lys-Leu-Ala-Leu-Gln-Lys-Tyr-Leu-Ala-

Ser-Ile-Leu-Gly-Ser-Arg-Thr-Ser-Pro-NH$_2$          (Ic)

The peptide of formula (Ia) is a shortened peptide [this peptide may hereinafter be abbreviated as "Hd-(1-31)-NH$_2$] which is composed of a sequence of the amino acids present in the region of from positon 1 to position 31 in the amino acid sequence of helodermin [which is totally composed of 35 amino acid residual groups]. The peptide of formula (Ib) is a shortened peptide [this peptide may hereinafter be abbreviated as Hd(1-32)-NH$_2$] which is composed of a sequence of the amino acids present in the region of from position 1 to position 32 in the amino acid sequence of helodermin. And the peptide of formula (Ic) is a shortened peptide [this peptide may hereinafter be abbreviated as "Hd(1-33)-NH$_2$] which is composed of a sequence of the amino acids present in the region of from position 1 to position 33 of the amino acid sequence of helodermin.

Further, according to a second aspect of the present invention, there is also provided a peptide represented by the following formula (II):

H-His-Ser-Asp-Ala-Val-Phe-Thr-Asp-Asn-Tyr-Thr-Lys

-Leu-Leu-Ala-Lys-Leu-Ala-Leu-Gln-Lys-Tyr-Leu-Ala-          (II)

Ser-Ile-Leu-Gly-Ser-Arg-Thr-NH$_2$

The peptide of formula (II) is a peptide [this peptide may hereinafter be abbreviated as "VIP(1-11)-Hd-(12-31)-NH$_2$] which is composed of a combination of a sequence of the amino acids present in the region of from position 1 [as located at the final N-terminal of VIP] to position 11 [this sequence may hereinafter be abbreviated as "VIP(1-11)-NH$_2$] amongst the 28 amino acid residues constituting the whole amino acid sequence of VIP, with a sequence of the amino acids present in the region of from position 12 to position 31 from the final N-terminal of helodermin [said sequence may hereinafter be abbreviated as "Hd-(12-31)-NH$_2$"].

In addition, according to a third aspect of the present invention, there is provided a peptide represented by the following formula (III):

```
H-His-Ser-Asp-Ala-Val-Phe-Thr-Asp-Asn-Tyr-Thr-Arg

-Leu-Arg-Lys-Gln-Met-Ala-Val-Lys-Lys-Tyr-Leu-Asn-        (III)

Ser-Ile-Leu-Asn-Ser-Arg-Thr-Ser-Pro-Pro-Pro-NH2
```

The peptide of formula (III) is a peptide [this peptide may hereinafter be abbreviated as "VIP(1-28)-Hd-(29-35)-NH$_2$] which is composed of a combination of the amino acid sequence of VIP [this sequence consists of the 28 amino acid residues and may hereinafter be abbreviated as "VIP(1-28)], with a sequence of the amino acids present in the region of from position 29 to position 35 from the final N-terminal of helodermin [said sequence may hereinafter be abbreviated as "Hd(29-35)"].

The peptides of the general formula (I), the peptide of formula (II) and the peptide of formula (III) each have activities of relaxing smooth muscle of blood vessel and of allowing the rate of blood stream to increase (hyperkinemic action), when tested with a canine femoral artery. These peptides can therefore be a hyperkinemic agent useful for human beings.

## BEST MODE FOR WORKING THE INVENTION

The individual peptides of formula (Ia), formula (Ib) and formula (Ic) which are included in the peptide of the general formula (I), as well as the peptide of formula (II) and the peptide of formula (III) according to this invention, all can be chemically synthesized by any conventional process for synthesis of peptides, either according to the solid phase method or according to the liquid phase method.

For instance, the novel peptides according to this invention can be chemically synthesized by any ordinary method known per se in the art, i.e., by a known active ester method or a known mixed anhydride method as described in many chemical textbooks and articles. It is however convenient to chemically synthesize the novel peptides by a solid-phase method where a resin bearing amino functions capable of immobilizing amino acids is employed.

Now, the chemical synthesis of the novel peptides of the present invention is illustrated with reference to some Examples. Incidentally, amino acids, amino acid residues, protected amino acids, amino-protecting groups, hydroxyl-protecting groups, activating groups and the like may sometimes be expressed by abbreviations as authorized by the IUPAC-IUB Commission on Biological Nomenclature, or by abbreviations commonly used in the field of the peptide synthesis.

Namely, abbreviations which are used in the subsequent Examples are as follows:

| | |
|---|---|
| Boc: | t-butyloxycarbonyl |
| Bzl: | benzyl |
| Tos: | p-toluenesulfonyl |
| ClZ: | 2-chlorobenzyloxycarbonyl |
| Cl$_2$•Bzl: | 2,6-dichlorobenzyl |
| OcHex: | cyclohexyl ester |
| DCC: | N,N-dicyclohexyl carbodiimide |
| HOBT: | 1-hydroxy benzotriazole |
| NHS: | N-hydroxysuccinimide |
| MA method: | Mixed anhydride method |
| AE method: | Active ester method |
| TLC: | thin layer chromatography |
| HPLC: | high performance liquid chromatography |
| TFA: | trifluoroacetic acid |
| NMM: | N-methylmorpholine |
| IBC: | iso-butylchloroformate |

4

Example 1

(a) Synthesis of the peptide of formula (Ic), namely, Hd(1-33)-NH$_2$ (by solid-phase synthetic method).

A benzhydrylamine-polystyrene resin (2.00g; a resin having a content of amino group of 0.5 mmol/g resin; purchased from Peptide Institute, Inc.) was used as an amino acid or peptide-immobilizing resin and charged in a reaction vessel of peptide synthesizer "Model 990C" which was manufactured by Beckman Instruments, Inc. The resin was stirred for 2 hours together with CH$_2$Cl$_2$ so that the resin was swollen.

Next, similarly to the procedures of the Step 5 to Step 10 described below, Boc-Pro-OH, namely Boc-protected derivative of proline, which is the amino acid present at position 33 of helodermin, was reacted with and combined with the resin, followed by making post-treatment.

Step 1: The protected peptide-resin combination as previously formed is washed three times with 20 mℓ of CH$_2$Cl$_2$.

Step 2: The protected peptide-resin combination is then pre-washed with 20 mℓ of 30% TFA solution in CH$_2$Cl$_2$.

Step 3: By further treatment with 20 mℓ of 30% TFA solution in CH$_2$Cl$_2$, the amino-protecting group present at the N-terminal amino acid of the peptide as bonded to the resin is removed from said N-terminal amino acid.

Step 4: The so deprotected peptide-resin combination is washed six times with 20 mℓ of CH$_2$Cl$_2$.

Step 5: The deprotected peptide-resin combination is then pre-washed with 20 mℓ of 7% N-methylmorpholine solution in CH$_2$Cl$_2$.

Step 6: The peptide-resin combination is subsequently neutralized with further 20 mℓ of 7% N-methylmorpholine solution in CH$_2$Cl$_2$.

Step 7: Washed six times with CH$_2$Cl$_2$.

Step 8: A solution, which was prepared by dissolving 3.0 mmol of a BOC-protected amino acid to be condensed newly and 3.0 mmol of HOBt in a mixed solvent of 7 mℓ of DMF and 7 mℓ of CH$_2$Cl$_2$, is added to the reaction vessel containing the peptide-resin.

Step 9: Seven milliliters of 0.5 M DCC solution in CH$_2$Cl$_2$ are added, followed by stirring the reaction mixture in the reaction vessel for 2 hours to conduct the coupling reaction of the Boc-protected amino acid.

Step 10: The reaction system is washed three times with 20 mℓ of CH$_2$Cl$_2$.

By conducting Step 5 to Step 10, the introduction of the amino acid Boc-Pro-OH into the resin was achieved at first so that BOC-protected proline was combined initially with the resin.

Starting from that the C-terminal Pro residue, namely the Pro residue which would be positioned at the C-terminal of the peptide to be synthetized was thus introduced into and combined with the resin as described above, predetermined protected amino acids were then successively introduced into and condensed with the peptide chain under construction which was fixed onto the resin. In each run for the condensation reaction of the respective amino acids, Steps 1 to 10 described above were repeated. The protected amino acids to be reacted were charged in the under-mentioned order, each in the amount of 3.0 mmol. Further, in each run of the condensation reaction, the amount of HOBt as added in step 8 was set at 3.0 mmol. Upon coupling of the final N-terminal His, however, HOBt was not used because His was susceptible to HOBt and could be modified in the imidazole moiety of its His molecule. The protected amino acids to be reacted subsequently to said first Boc-Pro-OH were added successively in the following order:

Boc-Ser(Bzl)-OH, Boc-Thr(Bzl)-OH,
Boc-Arg(Tos)-OH•4/5AcOEt•1/5H$_2$O,
Boc-Ser(Bzl)-OH, Boc-Gly-OH,
Boc-Leu-OH•H$_2$O, Boc-Ile-OH•1/2H$_2$O
Boc-Ser(Bzl)-OH, Boc-Ala-OH,
Boc-Leu-OH•H$_2$O, Boc-Tyr(Cℓ$_2$•Bzl)-OH,
Boc-Lys(Cℓ•Z)-OH, Boc-Gly-OH,
Boc-Leu-OH•H$_2$O, Boc-Ala-OH, Boc-Leu-OH•H$_2$O,
Boc-Lys(Cℓ•Z)-OH, Boc-Ala-OH,
Boc-Leu-OH•H$_2$O, Boc-Leu-OH•H$_2$O,
Boc-Lys(Cℓ•Z)-OH, Boc-Ser(Bzl)-OH,
Boc-Tyr(Cℓ$_2$•Bzl)-OH, Boc-Gln-OH,
Boc-Gln-OH, Boc-Thr(Bzl)-OH, Boc-Phe-OH,
Boc-Ile-OH•1/2H$_2$O, Boc-Ala-OH,

EP 0 450 100 A1

Boc-Asp(OcHex)-OH, Boc-Ser(Bzl)-OH,
Boc-His(Tos)-OH,

When the introduction and coupling of all the amino acids to be reacted was completed by the above procedures, there was synthesized on the resin such a protected peptide represented by the undermentioned formula, namely, a protected Hd(1-33).

```
Boc-His(Tos)-Ser(Bzl)-Asp(OcHex)-Ala-Ile-Phe-

Thr(Bzl)-Gln-Gln-Tyr(Cl₂·Bzl)-Ser(Bzl)-Lys(Cl·Z)-Leu-Leu-Ala-Lys(Cl·Z)-Leu-Ala-Leu-Gln-Lys(Cl·Z)-Tyr(Cl₂·Bzl)-Leu-Ala-Ser(Bzl)-Ile-Leu-Gln-

Ser(Bzl)-Arg(Tos)-Thr(Bzl)-Ser(Bzl)-Pro-NH-Resin.
```

The so synthesized protected peptide which was bound on the resin, namely, the protected peptide-resin combination as prepared in the above was then dried overnight in a desiccator. By taking 2.0g of the protected peptide-resin combination thus dried and treating it at 0°C for 1 hour with 20 mℓ of hydrogen fluoride in the presence of 2.0 mℓ of anisole, the removal of the amino-protecting groups from the peptide chain and the reaction for release of the deprotected peptide product out of the resin were performed. Hydrogen fluoride was then distilled off from the reaction mixture. After the residue was washed with ethyl ether and was well dried, the residue was mixed with 100 mℓ of 3 M acetic acid so that the peptide product was dissolved in the latter. Further, the insoluble resin was filtered off. The resulting filtrate containing the peptide product was lyophilized, whereby 892 mg of a crude peptide was obtained. This crude peptide was subjected to a desalting treatment by gel filtration, namely, by chromatography on a column of Sephadex G-25 (3 x 140 cm) as eluted with 3 M aqueous solution of acetic acid as eluent. Subsequently, the peptide was purified by a reverse-phase chromatography on a column of YMC Pack D-ODS-5 (2 x 25 cm) as eluted with 0.01 N HCl/CH₃CN solvent as eluent. A purified peptide Hd(1-33)-NH₂ was thus obtained in the manner described above and, after its acid hydrolysis, it showed the following amino acid analysis data:

Asp(1)0.98; Thr(2)1.91; Ser(5)4.55; Glu(3)3.01;

Gly(1)1.09; Ala(4)3.74; Ile(2)1.89; Leu(6)6.03;

Try(2)2.05; Phe(1)1.01; Lys(3)3.21; His(1)1.08:

Arg(1)0.90; Pro(1)0.99

(b) Synthesis of the peptide of formula (Ic), namely, Hd(1-33)-NH₂ (by liquid-phase synthetic method)

The six peptides (1) - (6) described below, which corresponded to the amino acid sequences in the region of positions 1 - 6, the region of positions 7 - 9, the region of positions 10 - 15, the region of positions 16 - 20, the region of positions 21 - 23 and the region of positions 24 - 33 of Hd(1-33), respectively, were synthesized by a peptide-synthesizing method commonly known in the art, according to the active ester method or the mixed anhydride method or the like. The individual peptides synthesized here were as follows:

(1) Boc-His(Tos)-Ser(Bzl)-Asp(OcHex)-Ala-Ile-Phe-OH
(2) Boc-Thr(Bzl)-Gln-Gln-OH
(3) Boc-Tyr(Cl₂·Bzl)-Ser(Bzl)-Lys(Cl-Z)-Leu-Leu-Ala-OH
(4) Boc-Lys(Cl·Z)-Leu-Ala-Leu-Gln-OH
(5) Boc-Lys(Cl·Z)-Tyr(Cl₂·Bzl)-Leu-OH
(6) Boc-Ala-Ser(Bzl)-Ile-Leu-Gly-Ser(Bzl)-Arg(Tos)-Thr(Bzl)-Ser(Bzl)-Pro-NH₂

Those six peptides were condensed together successively starting from the C-terminal peptide, namely, from the peptide (6) (an amidated derivative) by the fragment condensation method with making use of the

6

active ester method, so that a protected Hd(1-33)-NH$_2$ was obtained. The protected Hd(1-33)-NH$_2$ was treated in hydrogen fluoride in the presence of anisole to deprotect the protected Hd(1-33)-NH$_2$. Crude peptide Hd(1-33)-NH$_2$ so obtained was purified by gel filtration chromatography (on Sephadex G-25) and fractionating reverse-phase HPLC (on column: YMC Pack D-ODS-5), whereby a purified peptide Hd(1-33)-NH$_2$ was afforded.

Example 2

Synthesis of the peptide of formula (Ib), namely, Hd(1-32)-NH$_2$

Using the peptide synthesizer Model 990B as manufactured by Beckman Instruments, Inc., the corresponding amino acids as predetemined were successively condensed together by a solid-phase synthetic method in a manner similar to Example 1, to perform a peptide synthesis, whereby the peptide Hd(1-32)-NH$_2$ was obtained. In addition, a liquid-phase synthetic method was also conducted in a similar manner to Example 1 so that Hd(1-32)-NH$_2$ was again obtained.

An amino acid analysis of the peptide Hd(1-32)-NH$_2$ thus obtained was conducted after acid hydrolysis (with 6 N HCl, 110°C, 24 hours), thereby obtaining the following amino acid analysis data:

$$Asp(1)1.02; \quad Thr(2)2.01; \quad Ser(5)4.62; \quad Glu(3)3.03;$$

$$Gly(1)1.08; \quad Ala(4)4.17; \quad Ile(2)1.81; \quad Leu(6)5.94;$$

$$Try(2)1.91; \quad Phe(1)1.03; \quad Lys(3)3.30; \quad His(1)1.04;$$

$$Arg(1)0.98$$

Example 3

Synthesis of the peptide of formula (Ia), namely, Hd(1-31)-NH$_2$ according to the invention

Using the peptide synthesizer Model 990C as manufactured by Beckman Instruments, Inc., the corresponding amino acids predertermined were successively condensed together by a solid-phase method in a similar manner to Example 1 to perform a peptide synthesis, whereby the peptide Hd(1-31)-NH$_2$ was obtained. In addition, a liquid-phase method was also conducted in a similar manner to Example 1 so that Hd(1-31)-NH$_2$ was again obtained.

An amino acid analysis of the peptide thus obtained was conducted after acid hydrolysis (with 6 N HCl, 110°C, 24 hours), thereby obtaining the following amino acid analysis data:

$$Asp(1)1.08; \quad Thr(2)1.96; \quad Ser(4)3.76; \quad Glu(3)3.10;$$

$$Gly(1)1.13; \quad Ala(4)4.14; \quad Ile(2)1.90; \quad Leu(6)6.09;$$

$$Tyr(2)1.97; \quad Phe(1)1.09; \quad Lys(3)2.97; \quad His(1)0.98;$$

$$Arg(1)0.84$$

Example 4

Synthesis of the peptide of formula (II), namely, VIP(1-11)-Hd(12-31)-NH$_2$ according to the invention

The six peptides (1) - (6) identified below, which correspond to the amino acid sequences in the region of positions 1 - 4, the region of positions 5 - 9, the region of positions 10 - 15, the region of positions 16 - 20 the region of positions 21 - 23 and the region of positions 24 - 31 of the peptide VIP(1-11)-Hd(12-31)-NH₂, respectively, were synthesized by successively bonding the corresponding Boc-amino acids together by the stepwise elongation method. The condensation reaction of each Boc-amino acid was conducted using either the active ester method (AE method) or the mixed anhydride method (MA method). These six peptides synthesized here were as follows, which were each synthesized by the procedures described below.

(1) Boc-His(Tos)-Ser(Bzl)-Asp(OcHex)-Ala-OH
(2) Boc-Val-Phe-Thr(Bzl)-Asp(OcHex)-Asn-OH
(3) Boc-Tyr(Cℓ₂·Bzl)-Thr(Bzl)-Lys(Cℓ·Z)-Leu-Leu-Ala-OH
(4) Boc-Lys(Cℓ·Z)-Leu-Ala-Leu-Gln-OH
(5) Boc-Lys(Cℓ·Z)-Tyr(Cℓ₂·Bzl)-Leu-OH
(6) Boc-Ala-Ser(Bzl)-Ile-Leu-Gly-Ser(Bzl)-Arg(Tos)-Thr(Bzl)-NH₂

(1) Synthesis of Boc-His(Tos)-Ser(Bzl)-Asp(OcHex)-Ala-OH

In the presence of DCC as a condensing agent, 9.46g of Boc-Asp(OcHex)-OH (molecular weight: 315.37) and NHS (molecular weight: 115.09) were condensed together to give 14.05g of Boc-Asp(OcHex)-ONHS as an active amino acid ester (I) (yield: 85.02%). Next, condensation of 4.53g of the active amino acid ester (I) and 0.98g of H-Ala-OH (molecular weight: 89.11) by the active ester method (AE method) gave 3.26g of Boc-Asp(OcHex)-Ala-OH (molecular weight: 386.44) as a peptide (II) (yield: 76.8%). After 2.00g of the peptide (II) were de-butyloxycarbonylated by treatment with TFA, the resulting peptide, namely, Asp-(OcHex)-Ala-OH was condensed with 1.84g of Boc-Ser(Bzl)-OH (molecular weight: 295.33) by the mixed anhydride method (MA method), so that 2.54g of Boc-Ser(Bzl)-Asp(OcHex)-Ala-OH (molecular weight: 563.65) were obtained as a peptide (III) (yield: 87.1%). After 1.17g of the peptide (III) were de-butyloxycarbonylated by the treatment with TFA, the resulting peptide was condensed with 1.10g of Boc-His(Tos)-OH by the MA method, whereby 940mg of Boc-His(Tos)-Ser(Bzl)-Asp(OcHex)-Ala-OH (molecular weight: 854.98) were obtained as a peptide (IV) (yield: 53.0%).

(2) Synthesis of Boc-Val-Phe-Thr(Bzl)-Asp(OcHex)-Asn-OH

Condensation of 1.98g of Boc-Asp(OcHex)-ONHS with 1.98g of H-Asp-OH by the AE emthod gave 4.10g of Boc-Asp (OcHex)-Asn-OH as a peptide (V) (yield: 63.6%). After 3.87g of the peptide (V) were de-butyloxycarbonylated by the TFA treatment, the resulting peptide was condensed with Boc-Thr(Bzl)-OH by the MA method, so that 3.70g of Boc-Thr(bzl)-Asp(OcHex)-Asn-OH were obtained as a peptide (VI) (yield: 66.2%). After 3.70g of the peptide (VI) were de-butyloxycarbonylated by the TFA treatment, the resulting peptide was condensed with 2.21g of Boc-Phe-OH by the MA method, weereby 4.04g of Boc-Phe-Thr(Bzl)-Asp (OcHex)-Asn-OH were obtained as a peptide (VII) (yield: 88.3%). After 1.15g of the peptide (VII) were de-butyloxycarbonylated by the TFA treatment, the resulting peptide was condensed with 0.39g of Boc-Val-OH by the MH method, so that 0.78g of Boc-Val-Phe-Thr(Bzl)-Asp (OcHex)-Asn-OH was obtained as a peptide (VIII) (yeild: 60.0%).

(3) Synthesis of Boc-Tyr(Cl₂·Bzl)-Thr(Bzl)-Lys(Cl·Z)-Leu-Ala-OH

In the presence of DCC as a condensing agent, 11.56g of Boc-Leu-OH and 5.75g of NHS were condensed together to obtain 12.31g of Boc-Leu-ONHS as an active amino acid ester (IX) (yield: 75.0%). With 2.67g of H-Ala-OH by the AE method were condensed 9.85g of the active ester (IX), wereby 8.12g of Boc-Leu-Ala-OH were obtained as a peptide (X) (yield:89.3%). After 4.0g of the peptide (X) were de-butyloxycarbonylated by the TFA treatment, the resulting peptide was condensed with 4.88g of Boc-Leu-OH by the MH method, so that 3.72g of Boc-Leu-Leu-Ala-OH were obtained as a peptide (XI) (yield: 67.7g). Subsequent to de-butyloxycarbonylation of 2.50g of the peptide (XI) by the TFA treatment, the resulting peptide was condensed with 3.61g of Boc-Lys(Cl·Z)-OH by the MA method, affording 4.87g of Boc-Lys-(Cl·Z)Leu-Leu-Ala-OH as a peptide (XII) (yield: 94.0%). After 2.35g of the peptide (XII) was de-butyloxycarbonylated by the TFA treatment, the resulting peptide was condensed with 1.36g of Boc-Thr (Bzl)-OH, so that 2.15g of Boc-Thr(Bzl)-Lys(Cl·Z)-Leu-Leu-Ala-OH were obtained as a peptide (XIII) (yield: 72.1%). After 2.20g of the peptide (XIII) were de-butyloxycarbonylated by the TFA treatment, the de-butyloxycarbonylated derivative obtained was condensed with 1.40g of Boc-Tyr(Cl·Bzl)-OH by the MA method, so that 2.98g of Boc-Tyr(Cl₂·Bzl)-Thr(Bzl)-Lys(Cl·Z)-Leu-Leu-Ala-OH were obtained as a peptide (XIV) (yield: 99.8%).

(4) Synthesis of Boc-Lys(Cl·Z)-Leu-Ala-Leu-Gln-OH

Condensation of 3.28 g of Boc-Leu-ONHS with 1.46g of H-Gln-OH by the AE method gave 2.48g of Boc-Leu-Gln-OH as a peptide (XV) (yield: 69.1%). Subsequent to de-butyloxycarbonylation of 3.10g of the peptide (XV) by the TFA treatment, the resulting peptide was condensed with 2.12g of Boc-Ala-OH by the HA method to obtain 1.93g of Boc-Ala-Leu-Gln-OH as a peptide (XVI) (yield: 52.0%). After 900mg of the peptide (XVI) were de-butyloxycarbonylated by the TFA treatment, the resulting peptide was condensed with 680mg of Boc-Leu-OH by the HA method, whereby 710mg of Boc-Leu-Ala-Leu-Gln-OH were obtained as a peptide (XVII) (yield: 62.5%). Subsequent to de-butyloxycarbonylation of 670mg of the peptide (XVII) by the TFA treatment, the resulting peptide was condensed with 660mg of Boc-Lys(Cl•Z)-OH by the MA method. Boc-Lys (Cl•Z)-Leu-Ala-Leu-Gln-OH was thus obtained as a peptide (XVIII) in an amount of 850mg (82.1%).

(5) Synthesis of Boc-Lys(Cl•Z)-Tyr(Cl₂•Bzl)-Leu-OH

Boc-Tyr(Cl₂•Bzl)-OH (22.12g) and NHS (6.33g) were condensed together in the presence of DCC as a condensing agent, so that 15.84g of Boc-Tyr-(Cl₂•Bzl)-ONHS were obtained as a peptide (XIX) (yield: 58.7%). Condensation of 5.39g of the peptide (XIX) with 1.31g of H-Leu-OH by the AE method gave 3.93g of Boc-Tyr(Cl₂.Bzl)-Leu-OH as a peptide (XX) (yield: 71.1%). Subsequent to de-butyloxycarbonylation of 2.33g of the peptide (XX) by the TFA treatment, the resulting peptide was condensed with 2.09g of Boc-Lys (Cl•Z)-OH by the MA method. Boc-Lys(Cl•Z)-Tyr(Cl₂•Bzl)-Leu-OH was thus obtained as a peptide (XXI) in an amount of 2.56g (yield: 71.7%).

(6) Synthesis of Boc-Ala-Ser(Bzl)-Ile-Leu-Gly-Ser(Bzl)-Arg(Tos)-Thr-NH₂

To 6.19g of Boc-Thr(Bzl)-OH as a protected amino acid were added NMM and IBC in enquivalent amounts, respectively, to form a mixed acid anhydride. With the resulting mixed acid anhydride were reacted 2.53g of NHS, whereby the mixed acid anhydride was converted into an active amino acid ester. NH₃•H₂O was next added in a 10-fold amount to said ester to conduct an amidation reaction, so that 3.69g of Boc-Thr(Bzl)-NH₂ were obtained as amidated product (XXII) of the amino acid (yield: 59.8%). Subsequent to de-butyloxycarbonylation of 2.00g of the amidated product (XXII) by the TFA treatment, the resulting Thr-(Bzl)-NH₂ was condensed with 3.06g of Boc-Arg(Tos)-OH by the MA method to obtain 3.12g of Boc-Arg (Tos)-Thr(Bzl)-OH as a peptide (XXIII) (77.6%). After 2.16g of the peptide (XXIII) were de-butyloxycarbonylated by TFA treatment, the resulting Arg(Tos)-Thr(Bzl)-OH was condensed with 1.13g of Boc-Ser(Bzl)-OH, so that 2.29g of Boc-Ser(Bzl)-Arg(Tos)-Thr(Bzl)-NH₂ were obtained as a peptide (XXIV) (yield: 82.2%). Subsequent to de-butyloxycarbonylation of 2.00g of the peptide (XXIV) by TFA treatment, the resulting de-butyloxycarbonylated product was condensed with 0.87g of Boc-Leu-Gly-OH by the MA method to obtain 2.19g of Boc-Leu-Gly-Ser(Bzl)-Arg(Tos)-Thr(Bzl)-NH₂ as a peptide (XXV) (yield: 90.7%). The above Boc-Leu-Gly-OH had been obtained in the yield of 78.7% by condensing Boc-Leu-ONHS with H-Gly-OH in accordance with the AE method.

Next, 2.00g of the peptide (XXV) was treated with TFA to de-butyloxycarbonylate the same. Condensation of the de-butyloxycarbonylated product with 0.72g of Boc-Ile-OH by the MA method afforded 1.99g of Boc-Ile-Leu-Gly-Ser(Bzl)-Arg(Tos)-Thr(Bzl)-NH₂ as a peptide (XXVI) (yield: 89.1%). Subsequent to de-butyloxycarbonylation of 830mg of the peptide (XXVI) by TFA treatment, the resulting de-butyloxycarbonylated product was condensed with 270mg of Boc-Ser(Bzl)-OH by the MA method to give 630mg of Boc-Ser(Bzl)-Ile-Leu-Gly-Ser(Bzl)-Arg(Tos)-Thr(Bzl)-NH₂ as a peptide (XXVII) (yield: 66.8%). Subsequent to de-butyloxycarbonylation of 630mg of the peptide (XXVII) by TFA treatment, the resulting de-butyloxycarbonylated product was condensed with 110mg of Boc-Ala-OH by the MA method, so that 570mg of Boc-Ala-Ser(Bzl)-Ile-Leu-Gly-Ser(Bzl)-Arg(Tos)-Thr(Bzl)-NH₂ were obtained as a peptide (XXVIII) (yield: 85.9%).

(7) Synthesis of a protected peptide, VIP(1-11)-Hd(12-31)-NH₂

After 900mg of the peptide (XXVIII) obtained above in the procedure (6) were de-butyloxycarbonylated by TFA treatment, the resulting de-butyloxycarbonylated product was condensed with 693mg of the peptide (XXI) as obtained above in the procedure (5), by the DCC-HOBT method. The condensation product thus formed was post-treated by a method known per se in the art, followed by washing with methanol, so that 1.22g of Boc-Lys(Cl•Z)-Tyr(Cl₂•Bzl)-Leu-Ala-Ser(Bzl)-Ile-Leu-Gly-Ser(Bzl)-Arg(Tos)-Thr(Bzl)-NH₂ were obtained as a peptide (XXIX) (yield: 87.4%). After 1.00g of the peptide (XXIX) was de-butyloxycarbonylated by TFA treatment, the de-butyloxycarbonylated product was subjected to a fragment condensation with 490mg of the peptide (XVIII), which had been obtained above in the procedure (4), by the DCC-HOBt method.

Subsequent to dissolution of the resultant crude condensation product in DMF, the peptide product was reprecipitated from the resulting solution by the addition of AcOEt/Ether (1/4). As a peptide (XXX), 1.16g of Boc-Lys(Cl・Z)-Leu-Ala-Leu-Gln-Lys(Cl・Z)-Tyr(Cl$_2$・Bzl)-Leu-Ala-Ser(Bzl)-Ile-Leu-Gly-Ser(Bzl)-Arg(Tos)-Thr-(Bzl)-NH$_2$ were obtained (yield: 85.9%).

Subsequent to de-butyloxycarbonylation of 1.00g of the peptide (XXX) by TFA treatment, the resulting de-butyloxycarbonylated product was subjected to a fragment condensation with 662mg of the peptide (XIV), which had been obtained above in the procedure (3), by the DCC-HOBT method. The resultant crude condensation product was post-treated in the same manner as described above, whereby 1.39g of Boc-Tyr-(Cl$_2$・Bzl)-Thr(Bzl)-Lys (Cl・Z)-Leu-Leu-Ala-Lys(Cl・Z)-Leu-Ala-Leu-Gln-Lys(Cl・Z)-Tyr(Cl$_2$・Bzl)-Leu-Ala-Ser-(Bzl)-Ile-Leu-Gly-Ser(Bzl)-Arg (Tos)-Thr(Bzl)-NH$_2$ were obtained as a peptide (XXXI) (yield: 99.4%). After 600mg of the peptide (XXXI) were de-butyloxycarbonylated by TFA treatment, the resulting de-butyloxycarbonylated product was subjected to fragment condensation with 195 mg of the peptide (VIII), which had been obtained above in the procedure (2), by the DCC-HOBt method. The resulting crude condensation product was post-treated in the same manner as described above, whereby 530mg of Boc-Val-Phe-Thr(Bzl)-Asp(OcHex)-Asn-Tyr (Cl$_2$・Bzl)-Thr(Bzl)-Lys(Cl・Z)-Leu-Leu-Ala-Lys(Cl・Z)-Leu-Ala-Leu-Gln-Lys(Cl・Z)-Tyr-(Cl$_2$・Bzl)-Leu-Ala-Ser(Bzl)-Ile-Leu-Gly-Ser(Bzl)-Arg(Tos)-Thr(Bzl)-NH$_2$ were obtained as a peptide (XXXII) (yield: 74.1%). Subsequent to de-butyloxycarbonylation of 400mg of the peptide (XXXII) by TFA treatment, the resulting de-butyloxycarbonylated product was subjected to fragment condensation with 147mg of the peptide (IV), which had been obtained above in the procedure (1), by the DCC-HOBt method, so that 370mg of the protected VIP(1-11)-Hd(12-31)-NH$_2$ were obtained (yield: 79.8%).

(8) Synthesis of VIP(1-11)-Hd(12-31)-NH$_2$

After 200mg of the protected VIP(1-11)-Hd(12-31)-NH$_2$ obtained above in the procedure (7) were dissolved in 5mℓ of a (10:1) liquid mixture of anhydrous hydrogen fluoride and anisole, the resulting solution was stirred at 0°C fo 60 minutes to conduct the deprotecting reactions. The reaction solution was processed in a manner known per se in the art, followed by extraction with 3 M acetic acid. The extract obtained was lyophilized, and the resulting crude product was purified by a gel filtration chromatography (on Sephadex G-25) and reverse-phase HPLC (on YMC Pack D-ODS-5, as eluted with an eluent: 0.01 N hydrochloric acid-acetonitrile liquid mixture) to obtain 18mg of VIP(1-11)-Hd(12-31)-NH$_2$, namely, the peptide of formula (II) fo this invention.

An amino acid analysis (after acid hydrolysis with 6 N HCl, 110°C, 24 hours) of the peptide thus obtained gave the following amino acid analysis data:

Asp(3)2.88;  Thr(3)2.92;  Ser(3)2.78;  Glu(1)1.05;

Gly(1)1.07;  Ala(4)4.21;  Val(1)0.97;  Ile(1)1.02;

Leu(6)6.13;  Tyr(2)2.08;  Phe(1)0.95;  Lys(3)3.05;

His(1)1.01;  Arg(1)0.95

When this peptide was analyzed by HPLC, its elution time was 14.9 minutes. This HPLC was conducted under the following conditions: a column of YMC Pack R-ODS-5 (0.46 x 25 cm) was used and eluted gradiently with eluent of 0.01 N hydrochloric acid/acetonitrile (80/20) → (50/50), for gradient time of 30 minutes. Absorbance of UV at the wavelength of 210 nm was detected by a detector, and the sample solution was fed at the flow rate of 1.0 mℓ/min.

Example 5

Synthesis of the peptide of formula (II), namely, VIP(1-11)-Hd(12-31)-NH$_2$ of this invention (by solid-phase method)

Using the peptide synthesizer Model 990C as manufactured by Beckman Instruments, Inc., the corresponding amino acids as predetermined were successively condensed together by the solid-phase method in a similar manner to Example 1 to perform a peptide synthesis, whereby the peptide VIP(1-11)-

Hd(12-31)-NH$_2$ was obtained.

Example 6

Synthesis of the invention peptide of formula (III), namely, VIP(1-28)-Hd(29-35)-NH$_2$ (by solid-phase method)

In this Example, synthesis of the peptide VIP(1-28)-Hd(29-35)-NH$_2$ which is composed of the 35 amino acid residues was achieved by successively condensing the corresponding amino acids together in accordance with the solid-phase method and thereby extending the peptide chain stepwise in an automatic peptide synthesizer (Automatic Peptide Synthesizer Model 990B as manufactured by Beckman Instruments, Inc.).

As the solid-phase carrier for fixing the peptide under synthesis, a p-methylbenzhydrylamine-resin (1% divinylbenzene-polystyrene copolymer) (the -NH$_2$ content of 0.3 mmol/g-resin; 2.0 g) (a product of Kokusan Chemical Works) was placed in a reaction vessel of the automatic polypeptide synthesizer.

The amino-protecting group for blocking the $\alpha$-amino group of each amino acid to be condensed was t-butyloxycarbonyl group (Boc group). The Boc-amino acids employed as the starting materials were all purchased from Peptide Institute, Inc., Osaka.

Of those Boc-amino acids, Arg, Asp, His, Lys, Ser, Thr and Tyr, each of which is an amino acid containing a functional group in the side chain thereof, were used in the form of the following protected amino acids:

$$Boc-Arg(Tos)-OH, \quad Boc-Asp(OcHex), \quad Boc-His(Tos)-OH,$$

$$Boc-Lys(\varepsilon - 2-Cl-Z)-OH, \quad Boc-Ser(Bzl)-OH, \quad Boc-Thr$$

$$(Bzl)-OH, \quad Boc-Tyr(2,6-Cl_2-Bzl)-OH$$

All the protecting groups which were bonded to the side-chain functional groups of the above amino acids could remain stable during the condensation reactions of the amino acids but, after completed formation of the peptide chain, could be removed by treatment with liquefied hydrogen fluoride.

After the condensation of the individual amino acids, the N-terminal amino-protecting group, namely Boc group, of the peptide was removed. A trifluoroacetic acid (TFA) which was a de-butyloxycarbonylating reagent for that purpose was used in the form of a 50% TFA solution in CH$_2$Cl$_2$ (50% TFA/CH$_2$Cl$_2$).

Subsequent to the de-butyloxycarbonylating reaction, N,N-diisopropylethylamine (DIEA) was used in the form of 10% DIEA/CH$_2$Cl$_2$ as an agent for neutralizing the amino-group TFA salt as formed at the terminal of the peptide.

Dicyclohexylcarbodiimide (DCC) and 1-hydroxybenzotriazole (HOBt) were used as the condensing reagent for the condensation of the amino acids and as the condensation reaction catalyst, respectively.

Incidentally, all the solvents and reagents employed in this Example were highest grade regents or reagents of peptide-synthesizing grade.

When the condensation reaction of each amino acid was made, the progress of the reaction was checked by the Kaiser test. Thus, after the condensation reaction of each Boc-amino acid was made to its counterpart peptide chain as previously formed, about 0.01 g of the protected peptide-resin combination as produced in each run was samples into a test tube from the reaction vessel. The under-mentioned reagent solutions (1), (2) and (3) were added each in an amount of 3 droplets to the sample, followed by heating the resultant mixture for 2.5 minutes for 110$^\circ$C. When a positive reaction to ninhydrin was shown, the condensation reaction was resumed.

(1) A solution of 4 mg of phenol per m$\ell$ of ethanol,

(2) A solution as prepared by diluting 2 m$\ell$ of 1 mM KCN with 100 m$\ell$ of pyridine, and

(3) A solution of 50 mg of ninhydrin per m$\ell$ of ethanol.

The respective reaction steps conducted in this Example will hereinafter be described specifically.

(1) Synthesis of a protected peptide-resin combination

Added to the reaction vessel of an automatic peptide synthesizer were 2.0 g (0.6 mmol) of the p-methylbenzhydrylamine-resin (-NH$_2$ content of 0.3 mmol/g-resin), followed by stirring the resin in 45 m$\ell$ of

added DMF for 24 hours and then in 45 m$\ell$ of added $CH_2Cl_2$ for 24 hours to have the resin swollen. Using the predetermined corresponding Boc-amino acids one after another, condensation reactions for synthesis of the intended peptide were conducted.

Procedures required for effecting the introduction of each Boc-amino acid into the peptide chain included the step for removal of the Boc group from the N-terminal of the peptide as already formed on the resin, the step for neutralization, and the step for condensation of said Boc-amino acid. These procedures were all conducted in a dry nitrogen gas stream. These procedures were conducted by the following operations.

① The protected peptide-resin combination was placed in the reaction vessel and 40 m$\ell$ of $CH_2Cl_2$ were charged additionally. The resultant mixture was stirred for 1.5 minutes, followed by filtration. This procedure was repeated three times.

② The protected peptide-resin combination was then stirred for 1.5 minutes together with 40 m$\ell$ of 50% $TFA/CH_2Cl_2$ to perform the washing and equilibration of the resin, followed by filtration.

③ The protected peptide-resin combination was subsequently stirred together with 40 m$\ell$ of 50% $TFA/CH_2Cl_2$ for 30 minutes to effect the removal of the Boc group from the N-terminal of the peptide, followed by filtration.

④ The protected peptide-resin combination was stirred together with 40 m$\ell$ of $CH_2Cl_2$ for 1.5 minutes, followed by filtration. This procedure was repeated six times, whereby any TFA remaining on the peptide-resin combination was washed off.

⑤ 10% $DIEA/CH_2Cl_2$ (40 m$\ell$) was charged in the reaction vessel and stirred for 1.5 minutes together with the peptide-resin combination in said vessel, followed by filtration. This procedure was repeated three times to effect the neutralization of the TFA salt as formed in the peptide.

⑥ $CH_2Cl_2$ (40 m$\ell$) was charged into the reaction vessel and stirred for 1.5 minutes together with the peptide-resin combination in said vessel, followed by filtration. This procedure was repeated six times to eliminate any remaining DIEA.

⑦ Into the reaction vessel was charged a solution which had been prepared by dissolving three-fold equivalents (0.6 mmol) of the corresponding Boc-amino acid and HOBt in a mixture of 5 m$\ell$ of DMF and 10 m$\ell$ of $CH_2Cl_2$. The resultant mixture comprising said solution and the peptide-resin combination was stirred for 5 minutes to conduct equilibration of the resin with the reagents added.

⑧ 0.5 M $DCC/CH_2Cl_2$ (3.6 m$\ell$, 1.8 mmol of DCC) was added. The resulting mixture in the reaction vessel was stirred at room temperature for 120 minutes to effect the condensation reaction of the Boc-amino acid, followed by filtration.

⑨ $CH_2Cl_2$ (40 m$\ell$) was added to the produced peptide-resin combination, followed by stirring for 1.5 minutes and then by filtration. This procedure was repeated 6 times, so that any remaining reactive reagents were washed off.

After completion of the above mentioned series of operations, the reusltant protected peptide-resin combination as produced was stirred together with three 40 m$\ell$ portions of DMF each for 5 minutes, with three 40 m$\ell$ portions of MeOH each for 5 minutes and then with three 40 m$\ell$ portions of $CH_2Cl_2$ each for 5 minutes, successively in the order that these solvents are presented, so that the protected peptide-resin combination was washed. The latter was collected by filtration, on which the Kaiser test was then conducted. When a positive reaction to ninhydrin was shown, the above procedures (7) through (9) were repreated to complete the condensation reaction of each amino acid with the peptide chain.

By successively introducing the individual Boc-amino acids into the peptide and thus extending stepwise the peptide chain in the manner described above, a protected peptide containing the peptide VIP-(1-28)-Hd(29-35)-$NH_2$ was formed in such a state that said protected peptide was combined with and bonded on the resin. The protected peptide-resin combination was taken out of the reaction vessel, followed by successive washing with three 40 m$\ell$ portions of DMF, three 40 m$\ell$ portions of MeOH and three 40 m$\ell$ portions of $CH_2Cl_2$. The protected peptide-resin combination was collected by filtration and then dried under reduced pressure in a desiccator which contained $P_2O_5$ as a drying medium. As a result, the protected peptide-resin combination was obtained in a yield of 2.54 g.

(2) Treatment with liquefied hydrogen fluoride of the protected peptide-resin combination

The combination (1.27 g) of the resin with the protected peptide bound thereon, which had been obtained in the above procedure (1) and contained VIP(1-28)-Hd(29-35)-$NH_2$,was placed in an HF-reacting apparatus (manufactured by Peptide Institute, Inc., Osaka) together with TEFLON-coated stirring rods, followed by addition of 1.5 m$\ell$ of anisole and 0.5 m$\ell$ of ethyl methyl sulfide. The resultant mixture was left over at room temperature for 30 minutes under reduced pressure. While cooling the reaction vessel of said apparatus in a dry ice-acetone bath, redistilled hydrogen fluoride (15 mℓ) was added to the above-described mixture which was composed of the protected peptide-resin combination, anisole and ethyl methyl sulfide. The resulting mixture so formed was then stirred at 0°C for 60 minutes, whereby the peptide was released from the resin and concurrently the protecting groups for blocking the side-chain functional groups of the protected peptide were removed from the protected peptide chain. After HF was distilled off from the reaction mixture, the solid residue was washed three times with ethyl acetate (30 mℓ) and then collected by filtration. The solid residue was dried under reduced pressure and then extracted with 3 M acetic acid (100 mℓ) containing 0.02% ethanethiol so that an extract solution containing the peptide in a crude form was obtained. The resin was filtered off from the extract and the filtrate was lyophilized, whereby 455 mg of the crude peptide product was obtained.

(3) Tests of the crude peptide product

The purity of the crude peptide product as obtained above in the procedure (2) was determined by reverse-phase HPLC. An analytical, reverse-phase HPLC system as used included a high performance liquid chromatographic apparatus (TOSOH CCPD, manufactured by TOSOH CORPORATION), a low-pressure gradient apparatus GE-8000 (manufactured by TOSOH CORPORATION) and a flat pen recorder of Type 3066 (manufactured by YOKOGAWA ELECTRIC CORPORATION). YMC-Pack R-ODS-5 column (0.46 x 25 cm) (manufactured by YMC Company) was used as an eluting column. The elution conditions employed were such those that a mixture of 0.1 N HCℓ and $CH_3CN$ used as eluent was passed at a flow rate of 1.0 mℓ/minute and the ratio of 0.1 N HCl: $CH_3CN$ in the eluent was gradiently changed from 80:20 to 50:50 for 30 minutes. The peptide was detected by means of UV absorbance (at 210 mn).

A fraction having a principal elution peak of the crude peptide was collected. Where an amino acid analysis was made of an acid hydrolysate which resulted from acid hydrolysis of said crude peptide with 6 N HCl, the peptide thus obtained was found to have an amino acid composition in conformity with that of the intended VIP(1-28)-Hd(29-35)-$NH_2$.

(4) Purification of the crude peptide product

The crude peptide product as obtained in the above procedure (3) was then purified by a reverse-phase HPLC. A fractionating reverse-phase HPLC system used for this purification included a high performance liquid chromatographic apparatus LKB 2249 LC Gradient Pump (manufactured by Pharmacia LKB AB, Sweden), a ultraviolet and visible rays-spectrometric detector LKB 2251 (manufactured by Pharmacia LKB AB, Sweden), and a pen recorder LKB 2210 (manufactured by Pharmacia LKB AB, Sweden). A fractionating YMC-Pack D-ODS-5 column (2.0 x 25 cm) (manufactured by YMC Company) was used as an eluting column. A solution of the crude peptide product (50 mg) dissolved in 50% acetic acid was loaded on the chromatographic column, followed by elution with 0.01 N HCl/$CH_3CN$ at a flow rate of 10 mℓ/min. The eluate was collected in fractions and tested by menas of UV absorbance (at 210 nm), whereby such fractions containing the intended polypeptide were separated and collected. Those fracitons were lyophilized so taht 8.5 mg of a purified product of the intended polypeptide were obtained. The yield was 17% based on the crude peptide product.

(5) Amino acid analysis of the purified peptide

The purified peptide (80 μg) as obtained above in the procedure (4) was degasified and sealed in a hard glass tube in the presence of 0.3 mℓ of 6 N HCl containing phenol. After hydrolysis of the peptide at 110°C for 24 hours, the tube was opened and, over a boiling water bath, 6 N HCl was distilled off under reduced pressure out of the reaction solution from the hydrolysis. The resulting acid hydrolysate of the peptide was dissolved in 500 μℓ of lithium citrate buffer (product of Beckman Instruments, Inc.) and was then subjected to amino acid analysis by an automatic amino acid analyzer (Model: 7300, manufactured by Beckman Instruments, Inc.). The amino acid analysis data so obtained are given below and were in conformity with the amino acid composition of VIP(1-28)-Hd(29-35)-$NH_2$.

Asp(5)5.05; Thr(3)2.91; Ser(4)4.03; Glu(1)1.28;

Pro(3)3.31; Ala(2)2.03; Val(2)1.59; Met(1)0.99;

Ile(1)0.99; Leu(3)3.06; Tyr(2)1.87; Phe(1)0.80;

Lys(3)3.10, His(1)0.99; Arg(3)3.00

(6) Measurements of Rf value of the peptide in TLC and specific optical rotation

In silica gel TLC, the Rf value of the peptide which had been purified in the above procedure (5) was 0.00 when 1-BuOH-AcOH-$H_2$O(4:1:5) was used as a developer solvent (A), but 0.20 when 1-BuOH-pyridine-AcOH-$H_2$O (30:60:6:24) (upper phase) was used as a developer solvent (B).

Further, specific optical rotation, $[\alpha]_D^{21}$, of said peptide was -85° (c 0.1, 1 M acetic acid).

In addition, the Rf values of the peptide of this invention having formula (Ic) [Hd(1-33)-$NH_2$] as synthesized in Example 1 above, the peptide of this invention having formula (Ia) [Hd(1-31)-$NH_2$] as synthesized in Example 3 above and the peptide of this invention having formula (II) [VIP(1-11)-Hd(12-31)-$NH_2$] as synthesized in Example 4 above were similarly measured using the two types of the developer solvents (A) and (B). The specific optical rotations of these peptides were also measured. The results of measurements are summarized in the following table.

| Peptides of this Invention | Rf value (TLC, silica gel) | | Specific optical rotation |
|---|---|---|---|
| | Rf$^I$ | Rf$^{II}$ | |
| Hd(1-33)-$NH_2$ | 0.02 | 0.29 | |
| Hd(1-31)-$NH_2$ | 0.01 | 0.38 | |
| VIP(1-11)-Hd(12-31)-$NH_2$ | 0.13 | 0.45 | $[\alpha]_D^{25}$ -71° (c 0.1), 1 M acetic acid) |

Rf$^I$ : Rf values when developed by the developer solvent (A)

Rf$^{II}$: Rf values when developed by the developer solvent (B)

Physiological activities of the novel peptides according to the present invention were tested as described next.

Test Example

Influence of the peptides of this invention on the blood flow passing through canine femoral artery

The above-mentioned influence was estimated according to the VIP bioassay method proposed by Said and Mutt [see "Nature" 225, 863-864 (1970)]. After three mature beagles having the body weight of 10 kg were fasted for 18 hours, they were anesthetized with thiamylal (10 mg/kg) and Nembutal (25 mℓ/kg). During the experiment, physiological saline was continuously injected (2 mℓ/min.) into a vein of each beagle and the body temperature of the beagle was maintained at 37-38°C by an electric heater. The femoral artery was surgically exposed, and a polyethylene tube filled with heparin-added physiological saline was inserted in and fixed to a muscle branch of the femoral artery so that the polyethylene tube could be used as an injection route for each peptide under test. A probe of an electro-magnetic blood flowmeter was attached to the femoral artery on a peripheral side of the muscle branch so that the average flow rate of blood through the artery was recorded as time-dependent variations on a polygraphy.

A solution of 10 $\mu$g of each tested polypeptide dissolved in 25 $\mu\ell$ of 0.01 N HCl was diluted with physiological saline to prepare an injectable solution of the peptide. The injectable solution was injected into the artery by means of a peristaltic pump. Each peptide was injected at the dose of 200 p mol/kg. Tested peptides included the following compounds:

Compound 1:   Hd(1-33)-NH2 [the invention peptide of formula (Ic)]
Compound 2:   Hd(1-31)-NH$_2$ [the invention peptide of formula (Ia)]
Compound 3:   VIP(1-28)-Hd(29-35)-NH$_2$ [the invention peptide of formula (III)]
Compound 4:   VIP(1-11)-Hd(12-31)-NH$_2$ [the invention peptide of formula (II)]

Reference Compound: Helodermin

Upon elapsed times of 0.5 minute, 1 minute, 2 minutes, 3 minutes and 4 minutes after the injection of each peptide under test, the blood flow rate was measured. Increases in the femoral arterial blood flow rate (m$\ell$/min.) as comapred with the basal level of blood flow rate were calculated. The results of tests are summarized below in Table 1 and Table 2. It may be added that the test results of Table 1 and those of Table 2 were obtained through tests which were conducted by the same method but at different times.

## Table 1

| | Increase (m$\ell$/min.) in femoral arterial blood flow, as compared with the basal level of blood flow | | | | |
|---|---|---|---|---|---|
| Elapsed time (min.) | 0.5 | 1 | 2 | 3 | 4 |
| Compound 1 | 110 | 130 | 86 | 75 | 73 |
| Compound 2 | 185 | 112 | 41 | 40 | 44 |
| Helodermin (Reference) | 80 | 115 | 77 | 68 | 65 |

Table 2

| | Increase (mℓ/min.) in femoral arterial blood flow, as compared with the basal level of blood flow | | | | |
|---|---|---|---|---|---|
| Elapsed time (min.) | 0.5 | 1 | 2 | 3 | 4 |
| Compound 3 | 274 | 171 | 146 | 136 | 109 |
| Compound 4 | 271 | 99 | 75 | 65 | 62 |
| Helodermin (Reference) | 266 | 123 | 79 | 74 | 25 |

From the results of Table 1 and Table 2, the above Compounds 1, 2, 3 and 4, namely, the novel peptides of the present invention have been found to have an enhanced activity of increasing the blood flow rate over helodermin and have effects of sustaining the blood-flow-rate-increasing activity in comparison with helodermin.

INDUSTRIAL UTILITY OF THE INVENTION

As has been described above, novel peptides having effects of relaxing vascular smooth muscle and increasing the blood flow rate have now been provided by the present invention.

Claims

1. A peptide represented by the following general formula (I):

H-His-Ser-Asp-Ala-Ile-Phe-Thr-Gln-Gln-Tyr-Ser-Lys

-Leu-Leu-Ala-Lys-Leu-Ala-Leu-Gln-Lys-Tyr-Leu-Ala-          (I)

Ser-Ile-Leu-Gly-Ser-Arg-Thr-X-NH$_2$

wherein X means a bond (-), Ser or Ser-Pro.

2. Peptide represented by the following formula (II):

H-His-Ser-Asp-Ala-Val-Phe-Thr-Asp-Asn-Tyr-Thr-Lys

-Leu-Leu-Ala-Lys-Leu-Ala-Leu-Gln-Lys-Tyr-Leu-Ala-          (II)

Ser-Ile-Leu-Gly-Ser-Arg-Thr-NH$_2$

3. Peptide represented by the following formula (III):

H-His-Ser-Asp-Ala-Val-Phe-Thr-Asp-Asn-Tyr-Thr-Arg

-Leu-Arg-Lys-Gln-Met-Ala-Val-Lys-Lys-Tyr-Leu-Asn-    (III)

Ser-Ile-Leu-Asn-Ser-Arg-Thr-Ser-Pro-Pro-Pro-NH$_2$

# 国 際 調 査 報 告

国際出願番号PCT/JP90/01384

## I．発明の属する分野の分類

国際特許分類 (IPC)　Int. $Cl^5$

C07K7／10 , A61K37／02

## II．国際調査を行った分野

### 調 査 を 行 っ た 最 小 限 資 料

| 分 類 体 系 | 分 類 記 号 |
|---|---|
| IPC | C07K7／10 , A61K37／02 |

### 最小限資料以外の資料で調査を行ったもの

## III．関連する技術に関する文献

| 引用文献の<br>カテゴリー※ | 引用文献名　及び一部の箇所が関連するときは、その関連する箇所の表示 | 請求の範囲の番号 |
|---|---|---|
| X | Eur. J. Biochem ,第183巻,第2号(1989)<br>Jacques Abello , et. al「Properties of<br>vasoactive intestinal peptide receptors and<br>beta－adrenoceptors in the murine radiation<br>leukemia－virus－induced lymphoma cell line<br>BL／VL3」pp. 263－267 | 1－3 |
| X | Colloq. INSERM , 174 ( Forum Pept. , 2nd ,<br>1988 )(1989) P. Gourlet , et. al「Presence<br>of a new subclass of VIP receptors in human<br>SUP－T1 lymphoblasts」pp. 151－154 | 1－3 |
| X | FEBS Lett. ,第228巻,第2号(1988)<br>Patrick Robberecht , et. al「A new type of<br>functional VIP receptor has an affinity for<br>helodermin in human SUP－T1<br>lymphoblasts」pp. 351－355 | 1－3 |

※引用文献のカテゴリー

「A」特に関連のある文献ではなく、一般的技術水準を示すもの
「E」先行文献ではあるが、国際出願日以後に公表されたもの
「L」優先権主張に疑義を提起する文献又は他の文献の発行日
　　若しくは他の特別な理由を確立するために引用する文献
　　（理由を付す）
「O」口頭による開示、使用、展示等に言及する文献
「P」国際出願日前で、かつ優先権の主張の基礎となる出願の
　　日の後に公表された文献

「T」国際出願日又は優先日の後に公表された文献であって出
　　願と矛盾するものではなく、発明の原理又は理論の理解
　　のために引用するもの
「X」特に関連のある文献であって、当該文献のみで発明の新
　　規性又は進歩性がないと考えられるもの
「Y」特に関連のある文献であって、当該文献と他の1以上の
　　文献との、当業者にとって自明である組合せによって進
　　歩性がないと考えられるもの
「&」同一パテントファミリーの文献

## IV．認　　　証

| 国際調査を完了した日<br>05. 01. 91 | 国際調査報告の発送日<br>21.01.91 |
|---|---|
| 国際調査機関<br><br>日 本 国 特 許 庁 （ISA/JP） | 権限のある職員<br>特許庁審査官<br>前 田 憲 彦 ⊕     4H8318 |

| 第2ページから続く情報 | | |
|---|---|---|
| | **（Ⅲ欄の続き）** | |
| X | Peptides（Fayetteville，N.Y.），7（Suppl. 1）（1986）Patrick Robberecht，et. al 「Interaction of synthetic amino － and carboxy － terminal fragments of helodermin with rat liver VIP receptors」pp. 79－82 | 1－3 |
| X | Peptides（Fayetteville，N.Y.），7（Suppl.1）（1986）S. Naruse，et. al 「Helodermin has a VIP－like effect upon canine blood flow」pp. 237－240 | 1－3 |

**V. ☐ 一部の請求の範囲について国際調査を行わないときの意見**

次の請求の範囲については特許協力条約に基づく国際出願等に関する法律第8条第3項の規定によりこの国際調査報告を作成しない。その理由は、次のとおりである。

1. ☐ 請求の範囲＿＿＿＿＿は、国際調査をすることを要しない事項を内容とするものである。

2. ☐ 請求の範囲＿＿＿＿＿は、有効な国際調査をすることができる程度にまで所定の要件を満たしていない国際出願の部分に係るものである。

3. ☐ 請求の範囲＿＿＿＿＿は、従属請求の範囲でありかつPCT規則6.4(a)第2文の規定に従って起草されていない。

**VI. ☐ 発明の単一性の要件を満たしていないときの意見**

次に述べるようにこの国際出願には二以上の発明が含まれている。

1. ☐ 追加して納付すべき手数料が指定した期間内に納付されたので、この国際調査報告は、国際出願のすべての調査可能な請求の範囲について作成した。

2. ☐ 追加して納付すべき手数料が指定した期間内に一部分しか納付されなかったので、この国際調査報告は、手数料の納付があった発明に係る次の請求の範囲について作成した。
　　　請求の範囲＿＿＿＿＿＿＿

3. ☐ 追加して納付すべき手数料が指定した期間内に納付されなかったので、この国際調査報告は、請求の範囲に最初に記載された発明に係る次の請求の範囲について作成した。
　　　請求の範囲＿＿＿＿＿＿＿

4. ☐ 追加して納付すべき手数料を要求するまでもなく、すべての調査可能な請求の範囲について調査することができたので、追加して納付すべき手数料の納付を命じなかった。

追加手数料異議の申立てに関する注意
　　☐ 追加して納付すべき手数料の納付と同時に、追加手数料異議の申立てがされた。
　　☐ 追加して納付すべき手数料の納付に際し、追加手数料異議の申立てがされなかった。